# EUROPEAN PATENT SPECIFICATION

(11) **EP 0 646 643 B1**
(45) Date of publication and mention of the grant of the patent: **05.12.2001**
(21) Application number: 93110461.6
(22) Date of filing: 30.06.1993
(51) Int. Cl.: C12N 15/27, C12P 21/02, C12N 15/81, C12N 1/19, C07K 14/535

(54) **Modified human granulocyte macrophage-colony stimulating factor gene and expression thereof in yeast**
Modifizierte-Granulocyte-Makrophage-Kolonie-stimulierender-Faktor-Gen und seine Expression in Hefe
Gène humain modifié du facteur de stimulation des colonies de macrophages-granulocytes et son expression dans la levure

(43) Date of publication of application: 05.04.1995
(73) Proprietor: LUCKY LTD., Seoul 150-721 (KR)
(72) Inventor: Cho, Joong Myung, Seoul 134-080 (KR); Park, Young Woo, Daejeon 305-340 (KR)
(74) Representative: Grünecker, Kinkeldey, Stockmair & Schwanhäusser Anwaltssozietät

(56) References cited:
- EP-A- 0 337 359
- WO-A-89/03881
- DATABASE WPI Section Ch, Week 9302, 24 February 1992 Derwent Publications Ltd., London, GB; Class B04, AN 93-015736 CHO J. ; LEE Y. 'Human growth expression vector preparation involves inserting primers 1 and 2 into yeast c-DNA to synthesise specified gene' & KR-A-9 201 745 (LUCKY CO.) 24 February 1992
- JOURNAL OF BIOLOGICAL CHEMISTRY. (MICROFILMS), vol.257, no.6, 25 March 1982, BALTIMORE, MD US pages 3026 - 3031 J. F. BENNETZEN ET AL 'Codon selection in yeast'

## Description

### Field of the Invention

The present invention relates to a modified human granulocyte macrophage-colony stimulating factor("hGM-CSF") gene and an expression thereof; and, more specifically, relates to a modified hGM-CSF gene useful for the mass production of hGM-CSF in yeast, an expression vector containing said gene, a yeast cell transformed with said expression vector and a process for producing said hGM-CSF by employing yeast cells.

### Background of the Invention

Colony stimulating factor("CSF") refers to a family of lymphokines which induce progenitor cells to differentiate specific types of mature blood cells. A particuler type of mature blood cell that results from a progenitor cell depends upon the type of CSF present. Known types of CSF include G-CSF(Nicola et al., J. Biol. Chem. 258, 9017-9021(1983), M-CSF(Standley et al., J. Biol. Chem. 252, 4045-4052(1977), GM-CSF(Burgess et al., J. Biol. Chem. 252, 1991-2033(1977)) and multi-CSF(Burgess et al., Biochem. J. 185, 341-343(1980)).

The GM-CSF, which stimulates the formation of granulocyte-macrophage colony, is known to be produced in various cells such as epithelial cell, macrophage and lymphocyte and useful for the induction of hematopoietic cell's regeneration, and the treatment of exogenous infection and myeloid leukemia.

In 1985, cDNA clones encoding hGM-CSF were obtained by way of selecting them from the total cDNA clones prepared from the mRNA isolated from HUT-102 animal cells and T-lymphocyte cells by employing murine GM-CSF cDNA(Michael et al., P.N.A.S. 82, 6250-6254(1984)); and they were expressed to secrete hGM-CSF from a yeast cell by employing a yeast expression system containing an alpha factor promoter and an alpha factor leader sequence.

Meanwhile, Nicholas et al. and Gordon et al. have publiched respectively their expression results of a hGM-CSF gene in a COS-1 animal cell (Nicholas et al., EMBO 4, 645-653(1985); and Gordon et al., Science 228, 810-815(1985)).

On the other hand, GM-CSF includes two N-linked glycosylation recognition sites, i.e., asparagine-X-serine and asparagine-X-threonine(Asn-X-Ser and Asn-X-Thr, wherein X is any amino acid); and, therefore, is produced in a glycosylated form in an animal cell or yeast cell with a molecular weight ranging from 35 to 100Kd due to heterogenous N-glycosylation (Joachim et al., Bio/Technology 5, 831-834(1987)). Accordingly, it is difficult to purify the glycosylated GM-CSF produced in an animal or yeast cell in a high purity; and, therefore, the yield is poor.

In addition, it has been reported that a murine GM-CSF gene which has a nucleotide sequence homology of about 50% to human GM-CSF gene and also includes codons encoding two N-linked glycosylation recognition sites has been expressed in E. coli with the same biological activity as that of a natural murine GM-CSF even though the produced GM-CSF is not N-glycosylated(DeLamarter et al., EMBO 4, 2575-2581(1985)).

However, E. coli cells have a deficiency over yeast cells as a host cell in that the hGM-CSF produced in E. coli may be contaminated with E. coli native proteins such as endotoxin even after its purification, while yeasts do not produce any substance harmful to human, as borne out by the fact that yeasts have been used in the food processing industries for several centuries. Accordingly, the mass-production of non-N-glycosylated hGM-CSF in yeast cells is desirable.

In accordance with the present invention, therefore, it has now been found that a modified hGM-CSF gene, whose nucleotide sequence is modified to have yeast-preferred codons which are biased in strongly expressed genes of yeast such as glyceraldehyde-3-phosphate dehydroganase gene and alcohol dehydrogenase gene(Bennetzen, J.M. & Hall, B.D., J. Biol. Chem. 257, 3026(1982)) without changing the amino acid sequence encoded therein and to inactivate N-glycosylation sites by amino acid substitution while maintaining the biological activity of the hGM-CSF, can be expressed in yeasts to produce a high yield of the hGM-CSF with a nearly homogenous molecular weight.

From WO 89-03881 it is known how to construct a mutant amino acid sequence of human colony stimulating factor which is substantially homologous to the native sequence of hCSF, which has at least one N-glycosylation site, where the mutant sequence comprises at least one amino acid substitution, deletion or insertion inactivating the N-glycosylation site (on page 4, line 4 of the document, the deletion is disclosed as one of the possibilities to inactivate the N-glycosylation site). The cited non-glycosylated analogue of human GM-CSF is expressed and secreted by yeast at levels at several times greater than its recombinant native homologue.

An AG-Hybrid promotor is stated in the WPI database document (93-015736) which was used for the construction of a human growth hormone expression vector which involved the insertion of primers 1 and 2 into a yeast c-DNA to synthesise the specified gene.

### Summary of the Invention

Accordingly, it is an object of the present invention to provide a modified hGM-CSF gene which is designed to be efficient and useful for the mass-production of non-N-glycosylated hGM-CSF in yeast.

It is another object of the present invention to provide an expression vector comprising said hGM-CSF gene and a yeast cell transformed with the expression vector.

It is a further object of the present invention to provide a process for producing non-N-glycosylated hGM-CSF by employing the yeast transformant.

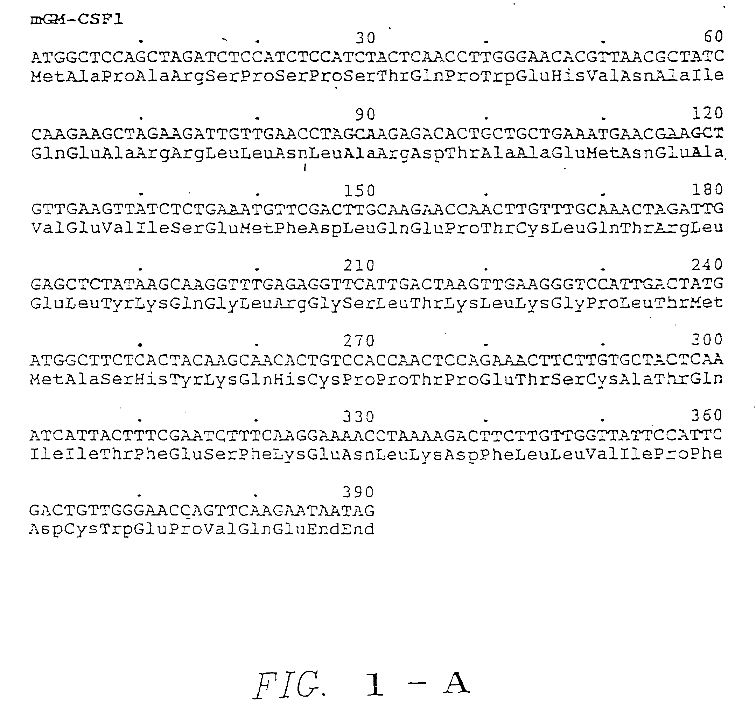

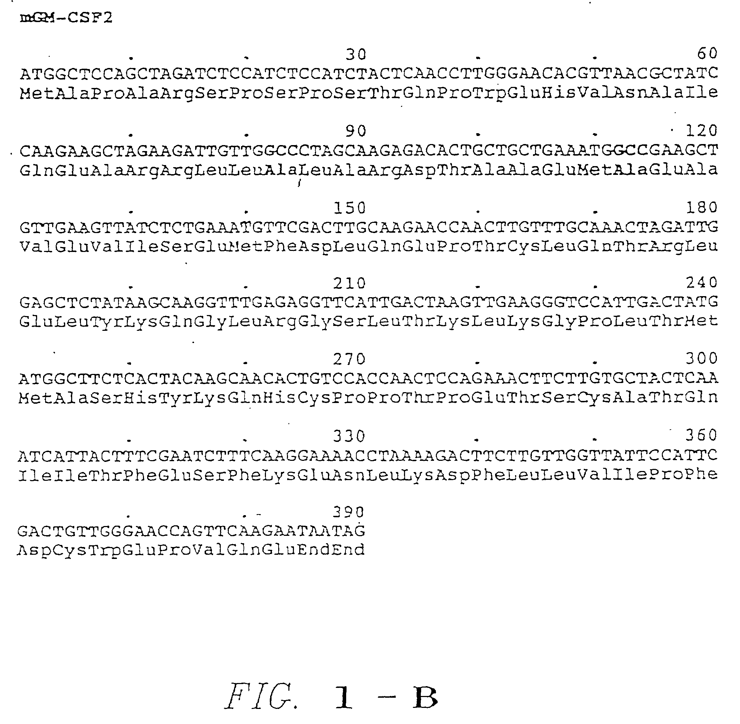

Accordingly, in accordance with one aspect of the present invention, there is provided a hGM-CSF gene having a nucleotide sequence shown in Fig. 1-a or 1-b which is designed to mass-produce non-N-glycosylated hGM-CSF in yeast.

In accordance with another aspect of the present invention, there is provided an expression vector comprising said hGM-CSF gene, and a yeast cell transformed with said vector.

In accordance with a further aspect of the present invention, there is provided with a process for mass-producing non-N-glycosylated hGM-CSF in yeast comprising culturing the yeast transformant under a culture condition appropriate for the expression of the hGM-CSF.

### Brief Description of the Drawings

Figures 1-a and 1-b represent the nucleotide sequence of the present modified hGM-CSF gene and the amino acid sequence derived therefrom, respectively.

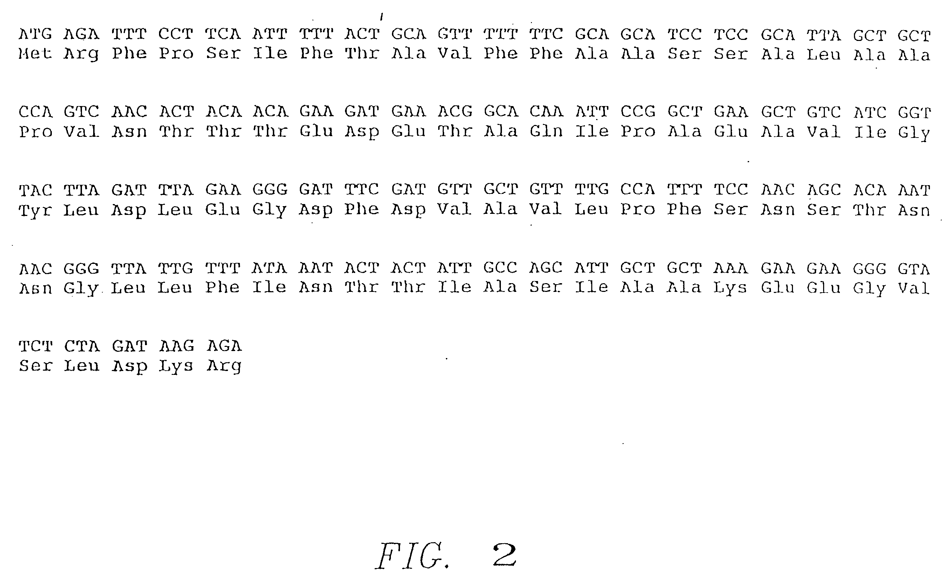

Figure 2 describes the nucleotide sequence encoding an alpha factor leader sequence and the amino acid sequence derived therefrom.

Figure 3 presents the strategy for constructing an expression vector comprising the hGM-CSF gene for the expression of the modified hGM-CSF gene in yeast.

Figure 4 shows the result of SDS-polyacrylamide gel electrophoresis of the yeast transformant cultured under the condition appropriate for the expression of the hGM-CSF gene.

### Detailed Description of the Preferred Embodiments

The modified hGM-CSF gene of the present invention designed to have yeast-preferred codons and to inactivate two N-linked glycosylation recognition sites has the nucleotide sequence of Fig. 1-a or 1-b, which may be chemically synthesized by employing a DNA synthesizer or by means of a site-directed mutagenesis (Kunkel, T.A., P.N.A.S. 82, 488(1985)) employing an already cloned hGM-CSF gene.

For example, the modified hGM-CSF gene may be obtained from a vector M13 mp18-GM-CSF(KFCC-10711; see Korean Patent Application No. 90-21308), which contains a hGM-CSF gene modified to have yeast-preferred codons(Bennetzen J.M. and Hall B.D., J. of Bio. Chem. 257, 3026-3031(1982)), by inactivating two N-linked glycosylation recognition sites of a hGM-CSF gene by substituting codons of the sites with other amino acid codons by employing site-directed mutagenesis, followed by digesting the resultant modified hGM-CSF gene with an appropriate restriction endonuclease(s).

The expression vector of the present invention useful for the expression of said hGM-CSF gene may be prepared by employing various expression systems operable in yeast. In accordance with a preferred embodiment, there is provided an expression vector prepared by cloning the modified hGM-CSF gene in a vector comprising an A/G hybrid promoter(see Korean Patent Appln. No. 89-20200 filed by the present applicant) which in prepared by connecting the "TATA box" of GAPDH(glyceraldehyde-3-phosphate dehydrogenase) promoter, one of the strongest yeast promoters, and the upstream activation sequence("UAS") of ADH2(alcohol dehydrogenase 2) promoter. Preferably, the modified hGM-CSF gene may be ligated with a DNA fragment encoding an alpha factor leader sequence since the alpha factor leader sequence preceding said hGM-CSF makes the latter be secreted into the culture medium and the leader sequence itself is cut out before the secretion, and the hGM-CSF can be easily purified from the medium.

For example, an expression vector to express the modified hGM-CSF gene of the present invention can be prepared by the following method:

Two primers AL1 and AL2 are synthesized to prepare a DNA fragment encoding the alpha factor leader sequence which is involved in the secretion system of proteins produced in yeast. Primer AL1 is designed to have a restriction endonuclease Bst BI recognition site for the ligation of the Bst BI recognition site located at the 3'-end of a hybrid A/G promoter; and primer AL2 is designed to have a restriction endonuclease XbaI recognition site at the position of 10 base pairs apart from the 3'-end of the leader sequence to facilitate the ligation with other DNA fragments. Using the above primers AL1 and AL2, the leader sequence of alpha factor having about 240 base pairs is amplified from the total nucleic acid of yeast(see, Janet Kurjan and Ira Herskowitz, Cell 30, 933-943(1982)).

The 3'-end region of the A/G promoter and 5'-end region encoding the alpha factor leader sequence digested by the restriction endonuclease Bst BI are ligated to obtain a fragment of about 1300 base pairs(fragment A), which is then digested with the restriction endonucleases BamHI and XbaI.

On the other hand, the M13mp18-GM-CSF modified to inactivate two N-glycosylation recognition sites by site specific mutagenesis is digested with the endonucleases XbaI and SalI to obtain a modified hGM-CSF gene fragment.

The fragment A containing the A/G promoter and the alpha factor leader sequence and the modified hGM-CSF fragment are cloned between the recognition sites of the restriction endonucleases BamHI and Sail of pYLBC-A/G-UB-HGH(ATCC 74071), an expression vector for yeast, to prepare a vector containing the modified hGM-CSF gene.

A yeast cell is transformed with the expression vector of the present invention by employing the conventional method (see, Beggs, Nature 275, 104(1978); and Hinnen et al., Proc. Natl. Aca. Sci. U.S.A. 75, 1929(1978)).

One of the resulting expression vectors in accordance with the present invention, which contains the modified hGM-CSF gene of Figure 1-a, was designated as LUCK-GM-CSF-1Y and the yeast transformed with the same(Saccharomyces cerevisiae LUCK-GM-CSF-1Y) was deposited with KFCC(Korean Federation of Culture Collections of Microorganisms) on December 12, 1990 with the accession number of KFCC-10716 and converted to the deposit under the Budapest Treaty on the International Recognition of the Deposit of Microorganisms for the Purpose of Patent Procedure on May 20, 1992 with the accession number of KCCM(Korean Culture Center of Microorganisms)-10028.

A yeast cell transformed with the expression vector containing the modified hGM-CSF gene is then cultured under a condition capable of expressing the gene, which is determined according to the characteristics of the vector and the host yeast cell. The non-N-glycosylated hGM-CSF produced in the yeast transformant or secreted into the culture medium may be isolated and purified by a combined use of conventional method, e.g., cell disruption, centrifugation, dialysis, salting out, chromatography, gel filtration, electrophoresis and electroelution.

The biological activity of the produced hGM-CSF can be measured by using the method of ELISA(enzyme linked immunoadsorbent assay).

The yield of the modified hGM-CSF is about 50µg/ml of the supernatant of the cell culture; and is higher than that known in the art.

The following Examples are intended to specifically illustrate the present invention without limiting its scope; and the experimental methods used in the Examples are practised in accordance with the Reference Examples given hereinbelow.

Unless otherwise specified, percentages given below for solids in solid mixtures, liquids in liquids and solids in liquids are on a wt/wt, vol/vol and wt/vol basis, respectively.

### Reference Example 1: Digestion of DNA with Restriction Endonuclease

The restriction endonucleases and reaction buffers used herein were purchased from NEB(New England Biolabs, U.S.A.).

The reaction was generally carried out in a 1.5ml sterilized Eppendorf tube with a reaction volume ranging from 50 to 100µl, at 37°C for 1 to 2 hours. Thereafter, the reaction mixture was heat-treated at 65°C for 15 minutes (or extracted with phenol and precipitated with ethanol in the case of a heat-resistant endonuclease) to inactivate the restriction endonuclease.

10 x reaction buffer for the reaction of a restriction endonuclease had the following composition:
10 x NEB reaction buffer 1: 100mM bis Tris propane-HCl, 100mM MgCl₂, 10mM dithiothreitol(DTT), pH 7.0;
10 x NEB reaction buffer 2: 100mM Tris-HCl, 100mM MgCl₂, 500mM NaCl, 10mM DTT, pH 7.0;
10 x NEB reaction buffer 3: 100mM Tris-HCl, 100mM MgCl₂, 10mM DTT, pH 7.0; and
10 x NEB reaction buffer 4: 100mM Tris-acetate, 100mM magnesium acetate, 500mM potassium acetate, 10mM DTT, pH 7.0.

### Reference Example 2: Phenol Extraction and Ethanol Precipitation

After the completion of the enzyme reaction, the reaction mixture was extracted with phenol for the purpose of inactivating the enzyme or recovering the DNA in the reaction mixture, wherein phenol preequilibrated with a buffer containing 10mM Tris-HCl (pH 8.0) and 1mM EDTA was used. Phenol extraction was carried out by mixing the equal volume of the sample and the phenol with vigorous shaking; centrifuging the mixture at 15,000rpm for 5 minutes; and transferring the aqueous layer into a new tube. The above procedure was repeated two or three times.

The aqueous layer was, then, extracted with an equal volume of chloroform solution(chloroform : isoamyl alcohol = 24:1) and the aqueous layer was separated again; 0.1 volume of 3M sodium acetate and 2.5 volumes of ethanol were added thereto; and, the mixture was centrifuged at 15,000rpm and 4°C for 20 minutes after having left it at -70°C for 30 minutes or at -20°C for 12 hours, to recover the nucleic acid.

### Reference Example 3: Ligation Reaction

Ligation of DNA was carried out by employing T₄ DNA ligase and 10 x ligation reaction buffer(0.5M Tris-HCl, 0.1M MgCl₂, 0.2M DTT, 10mM ATP, 0.5mg/ml bovine serum albumin (BSA)) purchased from NEB. The reaction volume was generally 20µl, and 10 units of T₄ ligase was used for the ligation of the cohesive ends of DNA while 100 units of T₄ ligase was used for the ligation of blunt ended DNAs.

The reaction was carried out at 16°C for 5 hours or at 4°C for over 14 hours; and, after the reaction was completed, the reaction mixture was heated at 65°C for 15 minutes to inactivate the T₄ DNA ligase.

### Reference Example 4: Transformation of E. coli

E. coli strains used for the Examples hereof include E. coli HB101(ATCC 33694), E. coli W3110(ATCC 27325), and E. coli JM105(ATCC 47016).

The host cells were incubated in 100ml of LB liquid medium(1% Bacto tryptone, 0.5% Bacto yeast extract, 0.5% NaCl) at 37°C until the O.D. (optical density) value at 650nm reached a range between 0.25 to 0.5. The cell culture was isolated by centrifugation and then washed with 50ml of 0.1M magnesium chloride(MgCl₂) solution. The cell pellet obtained by the centrifugation was added with 50ml of solution of 0.1M CaCl₂ and 0.05M MgCl₂ and then stood in an ice bath for 30 minutes. The cells were isolated by centrifugation and then suspended in 5ml of the solution of 0.1M CaCl₂ and 0.05M MgCl₂. The reagent and materials used above had been cooled to 0°C just before the use.

To 0.2ml of cell suspension obtained above was added 10µl of a ligation reaction mixture; and the reaction mixture was stood at 0°C for 40 minutes and then heated at 42°C for 1 minute. 2.0ml of LB liquid medium was added thereto; and the cells so treated were incubated at 37°C for 1 hour. The culture was then applied onto a Petri dish containing LB solid medium provided with an appropriate antibiotic such as ampicillin and incubated at 37°C overnight to obtain transformant colonies.

In the case of transformation of E. coli with M13 vector, M13 vector DNA(ligated with a gene) was added with 100µl of JM105 cell suspension, 15µl of 10mM isopropyl β-thioglactopyranoside, 25µl of 4% X-Gal and 3ml of 2 x YT soft solid medium(1% NaCl, 1% yeast extract, 1.6% Bactotryptone, 0.7% Bacto agar) preheated to 38°C. The mixture was stirred and applied onto 2x YT solid medium(1% NaCl, 1% yeast extract, 1.6% Bacto tryptone, 1.5% Bacto agar) and incubated at 37°C overnight to obtain plaques.

### Reference Example 5: Synthesis of Oligonucleotides

Oligonucleotides were synthesized by employing a DNA synthesizer(Applied Biosystems Inc., 380B, U.S.A.) of automatic solid phase phosphoamidite chemistry.

The synthesized oligonucleotides were purified by using a denaturing polyacrylamide gel(2M urea, 12% acrylamide and bis acrylamide(29:1), 50mM Tris, 50mM boric acid, 1mM EDTA-Na₂) electrophoresis and SEP-PAK(Waters Inc., U.S.A) column chromatography using a mixture of acetonitrile and water (50:50) as an eluent; and the amount was determined by measuring the O.D. value at 260nm.

### Example 1: Site-directed mutagenesis of hGM-CSF gene

### Step 1: Construction of primer

The two N-linked glycosylation sites of hGM-CSF(Asn-Leu-Thr and Asn-Glu-Ser) are present in the following 23rd to 42nd amino acid sequence of the hGM-CSF and are encoded in the following corresponding nucleotide sequence: In order to inactivate the two N-linked glycosylation sites, two primers CSF1 and CSF2 which have the following nucleotide sequence encoding the following amino acid sequences, respectively, were prepared:
CSF1 primer(used to substitute Ser and Thr of the two glycosylation sites with Ala, respectively) CSF2 primer (used to substitute Asn, Ser and Thr of the two glycosylation sites with Ala, respectively)

### Step 2: Site-directed mutagenesis

Using the two promers CSF1 and CSF2, the site-directed mutagenesis was carried out as follows:

1µl of E. coli CJ235.(Cat. # 170-3571, Bio-Rad Lab, U.S.A) was inoculated to 20ml of LB liquid medium containing 30µg/ml of chloramphenicol and cultured at 37°C for 6 hours. 10⁸ of M13mp18-CM-CSF M13 phage was inoculated to the culture and further cultured at 37°C overnight. After removing the cell pellet by centrifugation of the culture, to the supernatant so obtained was added 100µg of RNaseA; and the resultant was reacted for 30 minutes. A one-fifth volume of 20% PEG(polyethylene glycol) solution were added thereto to precipitate M13 phages; and the precipitates was dissolved in 100µl of TE solution(10mM Tris-HCl, 1mM EDTA, pH 7.5). Then the resulting solution was subjected to phenol or phenol/chloroform extraction and then ethanol precipitation to obtain the aqueous phase containing a single stranded nucleic acid of the phage. To a mixture of about 100ng of the nucleic acids so obtained, 2 to 3 pmoles of the primer CSF1 or CSF2 and 1µl of 10 x buffer solution(200mM Tris-HCl, pH 7.4, 20mM MgCl₂, 500mM NaCl) was added distilled water to make a total volume of 10µl; and the reaction mixture was heated at 70°C for 5 minutes and then cooled slowly to 30°C.

To the reaction mixture were added 1µl of 10 x buffer solution(5mM of each of dATP, dCTP, TTP and dGTP, 100mM Tris, pH 7.4, 50mM MgCl₂, 20mM DTT) and 1µl of T₄ DNA polymerase (1 unit/ml); and the resulting mixture was reacted in an ice-bath for 5 minutes, 25°C for 5 minutes and 37°C for 90 minutes in such order.

After the completion of the reaction, 90µl of TE solution kept at 90°C was added thereto and the mixture was stored at -20°C.

The E. coli JM105 was transformed with 2-3µl of the resultant mixture in accordance with the method of Reference Example 4.

Next day, a white plaque shown on YT medium was selected and cultured in the YT broth medium; and a replicative form(double stranded) DNA was extracted therefrom and digested with a restriction endonuclease PstI and electrophoresed to confirm the size of the digested DNA fragments. The plaque containing a DNA fragment of about 300bp length was selected to obtain the transformant containing the GM-CSF gene modified by the primer CSF1 or CSF2, which was designated as mGM-CSF1 or mGM-CSF2, respectively.

The nucleotide sequences thereof were determined by the Sanger's dideoxy method(P.N.A.S. 74, 5463(1977)) and shown in Figures 1-a and 1-b.

The reagent, enzyme and cell used herein were those taken from a Site-Directed Mutagenesis Kit(Cat. #170-3571) purchased from BIO-RAD Lab.

### Example 2: Isolation of total nucleic acids of yeast

Saccharomyces cerevisiae DCO4(Yeast Genetic Stock Center, University of California, Berkeley, CA, U.S.A) was cultured in 50ml of YEPD medium(1% yeast extract, 2% peptone, 2% glucose) for 24 hours and centrifuged at 5000rpm with Dynatec Clinical Centrifuge for 5 min to precipitate cells. The precipitated cells were dissolved in 10ml of 1M sorbitol and recentrifuged at 5000rpm for 5 min to obtain cells. To the precipitates were added 5ml of SD solution(1M sorbitol, 50mM potassium phosphate buffer solution, pH 7.5) and 5µl of 1M DTT. The reaction mixture was well mixed; and 1mg of zymolase(Miles Inc., U.S.A) was added thereto. The resulting mixture was maintained at 30°C for 30 minutes and centrifuged at 16,000 x G for 5 minutes to obtain the cell precipitates.

The resulting cell precipitates were dissolved in 5ml of TE buffer solution(1mM Tris, pH 7.5, 1mM EDTA), and then 0.5ml of 0.5M EDTA and 0.25ml of 10% SDS were added thereto. The reaction mixture was maintained at 65°C for 30 minutes and then 1ml of 5M potassium acetate was added thereto. The resulting mixture was reacted in an ice-bath for 1 hour and centrifuged at 20,000 x G for 25 minutes to isolate the supernatant. The supernatant was subjected to ethanol precipitation to obtain the total nucleic acid precipitate.

The precipitate was dried and redissolved in 50ml of TE buffer solution. To the obtained solution was added 20µl of RNase I (10mg/ml, BRL, U.S.A), and the resulting mixture was maintained at 37°C for 30 minutes.

The reaction mixture was extracted with phenol: chloroform:isoamyl alcohol(25:24:1 by volume) twice; and to the supernatant was added 2 volumes of ethanol to precipitate total nucleic acids.

The obtained precipitate was dissolved in 0.5ml of TE buffer solution for further use.

### Example 3: Preparation of a DNA fragment encoding alpha factor leader sequence and A/G hybrid promoter

The known alpha factor leader sequence which is involved in the secretion of proteins produced in yeast out of the cell(see, Cell 30, 933-943(1982)) was synthesized using PCR method.

The primer AL1 having the following nucleotide sequence was synthesized to link the alpha factor leader to the 3'-end of a hybrid A/G promoter having a restriction endonuclease Bst BI recognition site(5'-TTCGAA-3').

The primer AL2 having the following nucleotide sequence was synthesized to 23-mers containing a recognition site of restriction endonuclease XbaI and 3'-end of the alpha factor leader sequence-coding gene.

### Primer AL1:

### Primer AL2:

To 1µl of yeast DNA obtained from Example 2 above were added 10µl of 10 x Taq buffer solution (100mM Tris, pH 8.3, 500mM KC1, 15mM MgCl₂, 0.1% gelatin), 10µl of dNTP's(1.25mM of each of dGTP, dATP, TTP and dCTP), 2µg of primer AL1, 2µg of primer AL2, 68.5µl of distilled water and 0.5µl of Ampli Taq DNA polymerase(5 units/µl); and the resulting solution was thoroughly mixed. After adding 50µl of mineral oil thereto, the PCR reaction was carried out 25 times by using the thermal cycle of: 95°C for 30 seconds, 50°C for 30 seconds and 72°C for 1 minute, and the reaction was further carried out at 72°C for 10 minutes. The PCR product was subjected to phenol/chloroform extraction and ethanol precipitation; and the resultant precipitate was dissolved in 20µl of TE buffer solution. The obtained nucleic acid fragment was designated as "fragment A".

### Example 4: Preparation of expression vector for the secretion of GM-CSF from yeast.

### <Step 1>

About 1µg of fragment A obtained in Example 3 above was digested simultaneously with the restriction endonuclease Bst BI and XbaI under the condition of NEB buffer solution 4 to isolate a larger fragment which was designated as "fragment A-B/X".

On the other hand, M13mp18 DNA containing mGM-CSF1 (or mGM-CSF2) obtained in Example 1 above was digested with the restriction endonucleases XbaI and SalI to isolate about 410 bp fragment wherein 13 bp encoding-carboxy terminal region of alpha factor was ligated to mGM-CSF1(or mGM-CSF2), which was designated as "fragment mGM-CSF1-X/L(or mGM-CSF2-X/L)".

### <Step 2>

A plasmid pYLBC-A/G-UB-HGH(ATCC 74071) was digested with the restriction endonucleases PstI and SalI to obtain 9750 bp fragment containing the termination signal sequence of glyceraldehyde 3-phosphate dehydrogenase, which was named as "fragment PL". The plasmid was also digested with the restriction endonucleases PstI and Bst BI to obtain 3400 bp fragment, which was named as "fragment PB".

The ligation of the above two fragments was carried out as follows:

To a mixture of 2µl of 10 x ligation buffer solution (600mM Tris, pH 7.5, 10mM DTT, 100mM MgCl₂), 2µl of 10mM ATP, 1 unit of T₄ DNA ligase and 100ng of each of mGM-CSF1-X/L(or mGM-CSF2-X/L) and fragment A-B/X obtained in step 1 above was added distilled water to make a total volume of 20µl.

The reaction mixture was stood at 16°C for 6 hours. Thereafter, transformation of E. coli HB101(ATCC 33694) was performed using the ligation mixture in accordance with Reference Example 4. The transformant was selected on an ampicillin-containing agar plate, and the expression vector containing the alpha factor leader sequence and the modified hGM-CSF gene was isolated therefrom, which was designated as "Luck-GM-CSF-1Y(or Luck-GM-CSF-2Y)".

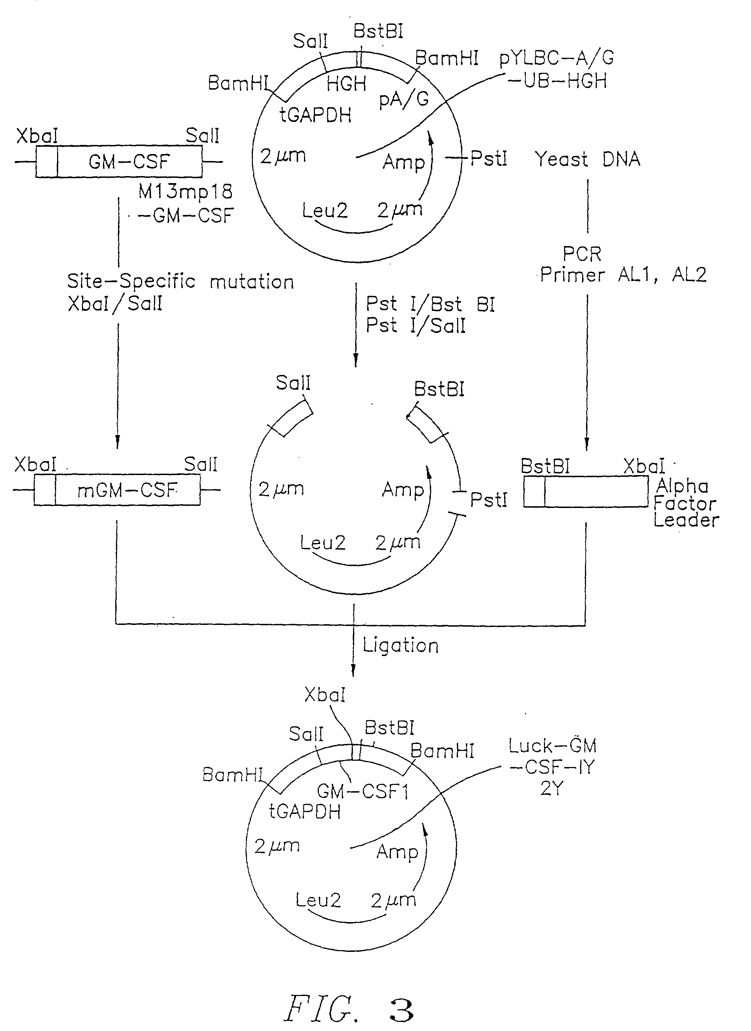

The nucleotide sequence of the vector isolated was confirmed by Sanger's dedioxy sequencing. The overall strategy to construct the above expression vector is shown in Figure 3.

### Example 5: The transformation of yeast with expression vector and the confirmation of expression by SDS-polyacrylamide gel

Saccharomyces cerevisiae DCO4(Yeast Genetic Stock Center, University of California, Berkeley, CA, U.S.A) was transformed with the expression vector obtained in Example 4 above in accordance with the method disclosed by Hinnen et al. (P.N.A.S. 79, 1929(1978)).

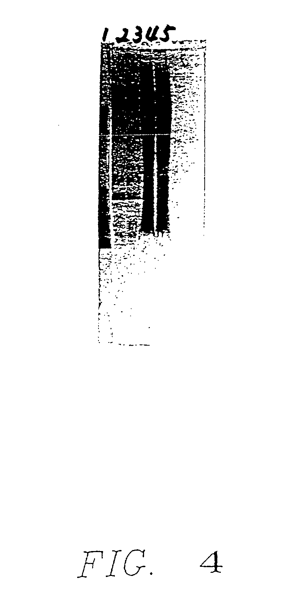

The transformant was selected on a leucine-deficient agar plate(6.7g of yeast nitrogen base without amino acids, 182g of sorbitol, 2% glucose, 0.25% Leu⁻ amino acid supplements, 20g of bacto agar), inoculated to 20ml of YEPD medium(2% peptone, 1% yeast extract, 2% glucose) and cultured at 30°C for 3 days. When the O.D. value at 650nm reached about 25, 1ml of the culture was taken and centrifuged to remove the yeast cells. To the resulting culture medium was added 250µl of 5-fold concentrated protein precipitating solution(50% trichloro acetate and 2% deoxycholate); and the resulting mixture was stood in an ice-bath for 10 minutes and centrifuged at 20,000 x G for 10min. The obtained precipitate was washed with 1ml of acetone, recentrifuged at 20,000 x G for 10 min and washed with acetone twice. The precipitate was dissolved in 20µl of TE buffer solution(10mM Tris-Cl, pH 7.5, 1ml EDTA); and the resulting mixture was poured into 10µl of 3-fold concentrated Laemmli sample buffer solution(Laemmli D.K., Nature 227, 680(1970)) and boiled at 100°C for 3 minutes. The resultant mixture was subjected to 15% SDS-polyacrylamide gel electrophoresis. The result is shown in Figure 4.

In Figure 4, lane 1 shows a standard protein size marker(Bio-Rad Lab., U.S.A) which represents the molecular weights of 43,000, 29,000, 18,400, 14,300, 6,200 and 3,000 dalton from the top, respectively; lane 2 shows the medium of a yeast cell culture containing the expression vector Luck-GM-CSF-1Y; lane 3 shows the medium of a yeast cell culture containing the expression vector Luck-GM-CSF-2Y; lane 4 shows an extract of yeast cell containing the expression vector Luck-GM-CSF-1Y; and lane 5 shows an extract of yeast cell containing the expression vector Luck-GM-CSF-2Y.

From the results of lanes 2 and 3 showing a dark band corresponding to the molecular weight of about 14,000, it is confirmed that the hGM-CSF protein having the nearly homogenous molecular weight was expressed in yeast cell and secreted into the culture medium(about 50µg hGM-CSF/ml of the culture medium). Also from the results of lanes 4 and 5, it is confirmed that the produced hGM-CSF protein remains in yeast cell with minor quantity.

Accordingly, the GM-CSF protein of the present invention can be expressed and secreted out of the yeast cell with higher yield than the original hGM-CSF gene.

Further, the biological activity of the hGM-CSF secreted from yeast cells was confirmed by the method of ELISA.

While the invention is described with respect to above specific emdobiments, it should be recognized that various modifications and changes which may be apparent to those skilled in the art to which the invention pertains may be made and also fall within the scope of the invention defined by the claims that follow.

## Claims

1. An expression vector comprising an A/G hybrid promoter having a TATA box of glyceraldehyde-3-phosphate dehydrogenase (GAPDH) promoter and an upstream activation sequence (UAS) of alcohol dehydrogenase 2(ADH2) promoter; and a modified human granulocyte macrophage-colony stimulating factor gene having the nucleotide sequence of SEQ ID NO: 5 or SEQ ID NO: 7.

2. The expression vector of claim 1 which is Luck-GM-CSF-1Y deposited under KCCM-10028.

3. A yeast cell transformed with the expression vector of claim 1 or 2.

4. The yeast cell of claim 3 which is the yeast cell deposited under KCCM 10028.

5. A process for producing a human granulocyte macrophage-colony stimulating factor which comprises culturing the yeast cell of claim 3 or 4.

## Patentansprüche

1. Expressionsvektor, umfassend einen A/G-Hybridpromotor mit einer TATA-Box des Glycerinaldehyd-3-phosphatdehydrogenase (GAPDH)-Promotors und einer stromaufwärts liegenden Aktivierungssequenz (UAS) des Alkoholdehydrogenase 2 (ADH2)-Promotors; und ein modifiziertes Gen von menschlichem Granulozyten-Makrophagen-Koionie-stimulierenden Faktor mit der Nukleotidsequenz von SEQ ID NO: 5 oder SEQ ID NO: 7.

2. Expressionsvektor nach Anspruch 1, bei dem es sich um Luck-GM-CSF-1Y handelt, der unter KCCM-10028 hinterlegt ist.

3. Hefezelle, die mit dem Expressionsvektor nach Anspruch 1 oder 2 transformiert ist.

4. Hefezelle nach Anspruch 3, bei der es sich um die Hefezelle handelt, die unter KCCM 10028 hinterlegt ist.

5. Verfahren zum Herstellen eines menschlichen Granulozyten-Makrophagen-Koloniestimulierenden Faktors, welches das Kultivieren der Hefezelle nach Anspruch 3 oder 4 umfasst.

## Revendications

1. Vecteur d'expression comprenant un promoteur hybride A/G ayant une boîte TATA de promoteur de glycéraldéhyde-3-phosphate déshydrogénase (GAPDH) et une séquence d'activation en amont (UAS) de promoteur d'alcool déshydrogénase 2 (ADH2) ; et un gène du facteur stimulant les colonies de granulocytes et macrophages humain modifié ayant la séquence nucléotidique SEQ ID NO:5 ou SEQ ID NO:7.

2. Vecteur d'expression selon la revendication 1, qui est Luck-GM-CSF-1Y déposé sous KCCM-10028.

3. Cellule de levure transformée avec le vecteur d'expression selon la revendication 1 ou 2.

4. Cellule de levure selon la revendication 3, qui est la cellule de levure déposée sous KCCM 10028.

5. Procédé pour produire un facteur de stimulation des colonies de granulocytes et macrophages humain, qui comprend la mise en culture de la cellule de levure selon la revendication 3 ou 4.


### SEQUENCE LISTING

(1) GENERAL INFORMATION:
   (i) APPLICANT:
      (A) NAME: Lucky Limited
      (B) STREET: 20, Yoido-dong, Yongdungpo-ku
      (C) CITY: Seoul
      (E) COUNTRY: Republic of Korea
      (F) POSTAL CODE (ZIP): 150-721
   (ii) TITLE OF INVENTION: MODIFIED HUMAN GRANULOCYTE MACROPHAGE-COLONY STIMULATING FACTOR GENE AND EXPRESSION THEREOF IN YEAST
   (iii) NUMBER OF SEQUENCES: 8
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 93110461.6
(2) INFORMATION FOR SEQ ID NO: 1:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: YES
   (iii) ANTI-SENSE: YES
   (ix) FEATURE:
      (A) NAME/KEY: conflict
      (B) LOCATION: replace(10..12, "")
      (D) OTHER INFORMATION: /note= "Ala coding sequence; modified sequence of GM-CSF"
   (ix) FEATURE:
      (A) NAME/KEY: conflict
      (B) LOCATION: replace(40..42, "")
      (D) OTHER INFORMATION: /note= "Ala coding sequence; modified sequence of GM-CSF"
   (xi) SEQUENCE DESCRIPTION: SEQ ID NO: 1:
   AACTTCAACA GCTTCGTTCA TTTCAGCAGC AGTGTCTCTT GCTAGGTTCA ACAATCTTCT 60
(2) INFORMATION FOR SEQ ID NO: 2:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: YES
   (iii) ANTI-SENSE: YES
   (ix) FEATURE:
      (A) NAME/KEY: conflict
      (B) LOCATION: replace(16..18, "")
      (D) OTHER INFORMATION: /note= "Ala coding sequence; modified sequence of GM-CSF"
   (ix) FEATURE:
      (A) NAME/KEY: conflict
      (B) LOCATION: replace(46..48, "")
      (D) OTHER INFORMATION: /note= "Ala coding sequence; modified sequence of GM-CSF"
   (xi) SEQUENCE DESCRIPTION: SEQ ID NO: 2:
   AACTTCAACA GTTTCGGCCA TTTCAGCAGC AGTGTCTCTT GATAGGGCCA ACAATCTTCT 60
(2) INFORMATION FOR SEQ ID NO: 3:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: YES
   (iii) ANTI-SENSE: NO
   (ix) FEATURE:
      (A) NAME/KEY: promoter
      (B) LOCATION: 1..30
      (D) OTHER INFORMATION: /note= "3' end sequence of GAPDH promoter"
   (ix) FEATURE:
      (A) NAME/KEY: misc_feature
      (B) LOCATION: 4..9
      (D) OTHER INFORMATION: /note= "Bst BI recognition site"
   (ix) FEATURE:
      (A) NAME/KEY: sig_peptide
      (B) LOCATION: 31..50
      (D) OTHER INFORMATION: /note= "5' end sequence of alpha factor"
   (xi) SEQUENCE DESCRIPTION: SEQ ID NO: 3:
   AGTTTCGAAT AAACACACAT AAACAAACAC CATGAGATTT CCTTCAATTT 50
(2) INFORMATION FOR SEQ ID NO: 4:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: YES
   (iii) ANTI-SENSE: NO
   (ix) FEATURE:
      (A) NAME/KEY: sig_peptide
      (B) LOCATION: 1..23
      (D) OTHER INFORMATION: /note= "3' end sequence of alpha factor"
   (ix) FEATURE:
      (A) NAME/KEY: misc_feature
      (B) LOCATION: 8..13
      (D) OTHER INFORMATION: /note= "Xba I recognition site"
   (xi) SEQUENCE DESCRIPTION: SEQ ID NO: 4:
   TCTCTTATCT AGAGATACTT CTT 23
(2) INFORMATION FOR SEQ ID NO: 5:
   (i) SEQUENCE CHARACTERISTICS:
      (A) LENGTH: 390 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: YES
   (iii) ANTI-SENSE: NO
   (vi) ORIGINAL SOURCE:
      (A) ORGANISM: Homo sapiens
      (H) CELL LINE: human HUT-102
   (ix) FEATURE:
      (A) NAME/KEY: CDS
      (B) LOCATION: 1..384
      (D) OTHER INFORMATION: /product= "modified granulocyte macrophage-colony stimulating factor" /standard_name= "modified hGM-CSF"
   (xi) SEQUENCE DESCRIPTION: SEQ ID NO: 5:
   ATG GCT CCA GCT AGA TCT CCA TCT CCA TCT ACT CAA CCT TGG GAA CAC        48
   Met Ala Pro Ala Arg Ser Pro Ser Pro Ser Thr Gln Pro Trp Glu His
     1               5                  10                  15
   GTT AAC GCT ATC CAA GAA GCT AGA AGA TTG TTG AAC CTA GCA AGA GAC        96
   Val Asn Ala Ile Gln Glu Ala Arg Arg Leu Leu Asn Leu Ala Arg Asp
                20                  25                  30
   ACT GCT GCT GAA ATG AAC GAA GCT GTT GAA GTT ATC TCT GAA ATG TTC       144
   Thr Ala Ala Glu Met Asn Glu Ala Val Glu Val Ile Ser Glu Met Phe
            35                  40                  45
   GAC TTG CAA GAA CCA ACT TGT TTG CAA ACT AGA TTG GAG CTC TAT AAG       192
   Asp Leu Gln Glu Pro Thr Cys Leu Gln Thr Arg Leu Glu Leu Tyr Lys
        50                  55                  60
   CAA GGT TTG AGA GGT TCA TTG ACT AAG TTG AAG GGT CCA TTG ACT ATG       240
   Gln Gly Leu Arg Gly Ser Leu Thr Lys Leu Lys Gly Pro Leu Thr Met
    65                  70                  75                  80
   ATG GCT TCT CAC TAC AAG CAA CAC TGT CCA CCA ACT CCA GAA ACT TCT       288
   Met Ala Ser His Tyr Lys Gln His Cys Pro Pro Thr Pro Glu Thr Ser
                    85                  90                  95
   TGT GCT ACT CAA ATC ATT ACT TTC GAA TCT TTC AAG GAA AAC CTA AAA       336
   Cys Ala Thr Gln Ile Ile Thr Phe Glu Ser Phe Lys Glu Asn Leu Lys
               100                 105                 110
   GAC TTC TTG TTG GTT ATT CCA TTC GAC TGT TGG GAA CCA GTT CAA GAA       384
   Asp Phe Leu Leu Val Ile Pro Phe Asp Cys Trp Glu Pro Val Gln Glu
           115                 120                 125
   TAATAG                                                                390
(2) INFORMATION FOR SEQ ID NO: 6:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Met Ala Pro Ala Arg Ser Pro Ser Pro Ser Thr Gln Pro Trp Glu His
    1               5                  10                  15
   Val Asn Ala Ile Gln Glu Ala Arg Arg Leu Leu Asn Leu Ala Arg Asp
               20                  25                  30
   Thr Ala Ala Glu Met Asn Glu Ala Val Glu Val Ile Ser Glu Met Phe
           35                  40                  45
   Asp Leu Gln Glu Pro Thr Cys Leu Gln Thr Arg Leu Glu Leu Tyr Lys
       50                  55                  60
   Gln Gly Leu Arg Gly Ser Leu Thr Lys Leu Lys Gly Pro Leu Thr Met
   65                  70                  75                  80
   Met Ala Ser His Tyr Lys Gln His Cys Pro Pro Thr Pro Glu Thr Ser
                   85                  90                  95
   Cys Ala Thr Gln Ile Ile Thr Phe Glu Ser Phe Lys Glu Asn Leu Lys
               100                 105                 110
   Asp Phe Leu Leu Val Ile Pro Phe Asp Cys Trp Glu Pro Val Gln Glu
           115                 120                 125
(2) INFORMATION FOR SEQ ID NO: 7:
   (i) SEQUENCE CHARACTERISTICS:
      (A) LENGTH: 390 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: YES
   (iii) ANTI-SENSE: NO
   (vi) ORIGINAL SOURCE:
      (A) ORGANISM: Homo sapiens
      (H) CELL LINE: human HUT-102
   (ix) FEATURE:
      (A) NAME/KEY: CDS
      (B) LOCATION: 1..384
      (D) OTHER INFORMATION: /product= "modified granulocyte macrophage-colony stimulating factor" /standard_name= "modified hGM-CSF"
   (xi) SEQUENCE DESCRIPTION: SEQ ID NO: 7:
(2) INFORMATION FOR SEQ ID NO: 8:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO: 8:
   Met Ala Pro Ala Arg Ser Pro Ser Pro Ser Thr Gln Pro Trp Glu His
   1               5                   10                  15
   Val Asn Ala Ile Gln Glu Ala Arg Arg Leu Leu Ala Leu Ala Arg Asp
               20                  25                  30
   Thr Ala Ala Glu Met Ala Glu Ala Val Glu Val Ile Ser Glu Met Phe
           35                  40                  45
   Asp Leu Gln Glu Pro Thr Cys Leu Gln Thr Arg Leu Glu Leu Tyr Lys
       50                  55                  60
   Gln Gly Leu Arg Gly Ser Leu Thr Lys Leu Lys Gly Pro Leu Thr Met
   65                  70                  75                  80
   Met Ala Ser His Tyr Lys Gln His Cys Pro Pro Thr Pro Glu Thr Ser
                   85                  90                  95
   Cys Ala Thr Gln Ile Ile Thr Phe Glu Ser Phe Lys Glu Asn Leu Lys
               100                 105                 110
   Asp Phe Leu Leu Val Ile Pro Phe Asp Cys Trp Glu Pro Val Gln Glu
           115                 120                 125